(19) Europäisches Patentamt
European Patent Office
Office européen des brevets

(11) **EP 4 324 455 A1**

(12) **EUROPEAN PATENT APPLICATION**
published in accordance with Art. 153(4) EPC

(43) Date of publication:
**21.02.2024 Bulletin 2024/08**

(21) Application number: **22787532.5**

(22) Date of filing: **12.04.2022**

(51) International Patent Classification (IPC):
**A61K 9/12** (2006.01)      **A61M 11/00** (2006.01)

(52) Cooperative Patent Classification (CPC):
**A24B 15/167; A61K 9/12; A61M 11/00**

(86) International application number:
**PCT/CN2022/086384**

(87) International publication number:
**WO 2022/218310 (20.10.2022 Gazette 2022/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.04.2021 CN 202110398742**

(71) Applicant: **Shenzhen First Union Technology Co., Ltd.**
**Shenzhen, Guangdong 518000 (CN)**

(72) Inventors:
• **LU, Jin**
  **Shenzhen, Guangdong 518000 (CN)**
• **XU, Zhongli**
  **Shenzhen, Guangdong 518000 (CN)**
• **LI, Yonghai**
  **Shenzhen, Guangdong 518000 (CN)**

(74) Representative: **Proi World Intellectual Property GmbH**
**Obermattweg 12**
**6052 Hergiswil, Kanton Nidwalden (CH)**

(54) **AEROSOL-FORMING SUBSTRATE AND AEROSOL-GENERATING SYSTEM**

(57)    An aerosol-forming substrate and an aerosol generation system are provided. The aerosol-forming substrate includes yohimbine or a pharmaceutically acceptable salt of yohimbine or yohimbine hydrate. Based on a total mass of the aerosol-forming substrate, a mass percent of the yohimbine or the pharmaceutically acceptable salt of the yohimbine or the yohimbine hydrate is in a range of 0.1%-10%, preferably 0.1%-8%, further preferably 0.1%-6%, further preferably 0.1%-5%, further preferably 0.5%-5%, further preferably 1%-5%, and further preferably 2%-5%. In the aerosol-forming substrate containing yohimbine or a pharmaceutically acceptable salt of yohimbine or yohimbine hydrate provided in this application, no carbonization occurs after repeated heating by using an electronic vaporization device, and a conversion rate of yohimbine is relatively high, so that a content of yohimbine is close to a dosage through oral administration or injection in current clinical trials, thereby guaranteeing safety.

FIG. 1

**Description**

**CROSS-REFERENCE TO RELATED APPLICATIONS**

This application claims priority to Chinese Patent Application No. 202110398742.4, filed with the China National Intellectual Property Administration on April 12, 2021 and entitled "AEROSOL-FORMING SUBSTRATE AND AEROSOL GENERATION SYSTEM", which is incorporated herein by reference in its entirety.

**TECHNICAL FIELD**

This application relates to the technical field of vaporization, and in particular, to an aerosol-forming substrate and an aerosol generation system.

**BACKGROUND**

Yohimbine is an alkaloid of natural origin. The ingredient was first extracted from the bark of the Rubiaceae Corunant Yohimbine tree in West Africa. The chemical formula for yohimbine is methyl 17alpha-hydroxyyohimban-16alpha-carboxylate, and the molecular formula is $C_{21}H_{26}N_2O_3$.

Literature research shows that yohimbine has a wide range of pharmacological effects and has been developed for clinical treatment of diseases such as arteriosclerosis and rheumatism. The most obvious pharmacological effect is to treat male sexual dysfunction.

At present, most clinical trials are administered orally or by injection. Studies have proved that yohimbine is a one-compartment metabolic model. After a single oral administration, the metabolism in vivo is boosted, and Tmax and t1/2 are both less than 1h. Since the oral bioavailability is very low and the difference is obvious, the average value is from 22.3% to 33%. Therefore, it is necessary to develop inhalation and administration routes to improve bioavailability.

During implementation of this application, the inventor found that: the patent document with the Application Publication No. of CN112155255A discloses that an evaporable material may contain plant medicines and/or nutrients, for example: yohimbine. In some implementations, the evaporable material is at least 50% soluble (by weight) in any suitable carrier solvent. The solvent is, for example, glycol (such as propanediol and vegetable glycerol), ethylene glycol, dipropylene glycol, trimethylene glycol, ethanol, and a combination thereof.

The disclosed solution has the problems that, on the one hand, the solubility of yohimbine in the carrier solvent is poor, and at least 50% (by weight) of yohimbine cannot be completely dissolved; whether the evaporable material containing the weight can be evaporated and the influence on an electronic evaporator device cannot be verified from this document; and even if the evaporable material can be evaporated, the conversion rate thereof is also very low. On the other hand, the dosage of the evaporable material containing at least 50% (by weight) yohimbine (even if the conversion rate is very low) is very large for users, far exceeding the dosage of administration orally or by injection in present clinical trials, and the safety cannot be guaranteed.

**SUMMARY**

During implementation of this application, the inventor finds that a first aspect of this application is intended to provide an aerosol-forming substrate with little influence on an electronic vaporization device, a high conversion rate, and guaranteed safety. The aerosol-forming substrate includes yohimbine or a pharmaceutically acceptable salt of yohimbine or yohimbine hydrate.

Based on a total mass of the aerosol-forming substrate, a mass percent of the yohimbine or the pharmaceutically acceptable salt of the yohimbine or the yohimbine hydrate is in a range of 0.1%-10%, preferably 0.1%-8%, further preferably 0.1%-6%, further preferably 0.1%-5%, further preferably 0.5%-5%, further preferably 1%-5%, and further preferably 2%-5%.

In some implementations, the pharmaceutically acceptable salt is selected from at least one of the following: hydrochloride, hydrobromide, benzoate, hydrofluoride, sulfate, nitrate, phosphate, formate, acetate, propionate, oxalate, malonate, succinate, fumarate, maleate, lactate, malate, tartrate, citrate, picrate, mesylate, esilate, benzene sulfonate, aspartate, and glutamate.

In some implementations, the pharmaceutically acceptable salt includes benzoate.

In some implementations, the aerosol-forming substrate further includes a solvent, where the solvent is selected from at least one of the following:

propanediol, butanediol, pentanediol, hexanediol, 1,2,4-butanetriol, dipropylene glycol, diethylene glycol, triethylene glycol, polyethylene glycol 200, polyethylene glycol 400, glycerol triacetate, glycerol, dipropylene glycol ether, ethanol, water, triethyl citrate, caprylic capric triglyceride, isopropanol, sweet orange oil, lemon oil, peppermint oil, palm oil, peanut oil, corn oil, salad oil, xylitol, sorbitol, and erythritol.

In some implementations, the solvent includes propanediol, and a mass percent of the propanediol in the aerosol-forming substrate is in a range of 20%-60%, preferably 20%-50%, further preferably 20%-45%, further preferably 25%-45%, further preferably 30%-45%, and further preferably 35%-45%.

In some implementations, the solvent includes glycerol, and a mass percent of the glycerol in the aerosol-forming substrate is in a range of 10-60%, preferably 10%-50%, further preferably 10%-40%, further preferably 20%-40%, and further preferably 30%-40%.

In some implementations, a mass ratio of the propanediol to the glycerol is greater than 1, preferably 1.1-4:1, and further preferably 2-4:1.

In some implementations, the aerosol-forming substrate further includes a flavoring agent.

The flavoring agent in the aerosol-forming substrate is added according to the conventional amount required for flavoring, and the mass percent is in a range of 1-20%, preferably 5%-20%, further preferably 10%-20%, and further preferably 15%-20%. The flavoring agent is selected from at least one of the following:

nerol, trans-2-hexenol, linalool, benzyl alcohol, 1-hexanol, leaf alcohol, alpha-terpineol, citronellol, beta-phenylethyl alcohol, linalool oxide, geraniol, isoamyl alcohol, octanol, hexanol, decyl alcohol, cinnamyl alcohol, heptanol, eugenol, maltol, ethyl maltol, thymol, isoeugenol, 2-methylbutyric acid, malic acid, n-valeric acid, hexanoic acid, edible acetic acid, n-caprylic acid, strawberry acid, butyric acid, citric acid, propionic acid, 3-methylvaleric acid, isovaleric acid, isoamyl acetate, amyl formate, geranyl formate, butyl formate, benzyl formate, cis-3-Hexenyl formate, gamma-Decanolactone, delta-Nonalactone, gamma-Octalactone, gamma-Heptalactone, gamma-Unsecalactone, delta-Dodecalactone, benzaldehyde, strawberry aldehyde, cinnamyl aldehyde, furfural, citral, acetaldehyde, 3-(Methylthio)propionaldehydel, 3-Mercapto-2-methylpentanal, isobutyraldehyde, trans-2-Octenal, trans-2-Nonenal, trans-2-Decenal, trans,trans-2,4-heptadienal, 2,5-Dimethyl pyrazine, 2-Acetylfuran, 2-Ethyl-3,(5 or 6)-dimethylpyrazine, 2,3,5,6-Tetramethylpyrazine, 2,3,5-Trimethylpyrazine, 2-acetyl pyrazine, acetophenone, beta-ionone, Damascenone 2 (Firmenich), butanedione, alpha-ionone , acetoin (acetyl methyl carbinol), acetoin (acetyl methyl alcohol), methyl heptenone, vanillin, ethyl vanillin, dihydrocoumarin, raspberry ketone, anisole, cedryl methyl ether, methyl 2-methyl-3-furyl disulfide, wintergreen oil, clove bud oil, 10-fold sweet orange oil, rosewood oil, geranium oil, bitter almond oil, clove basil oil, ethyl vanillin, dihydrocoumarin, raspberry ketone, trans-2-ethoxy-5-(1-propenyl)phenol, tolu concrete, Peru concrete, oak concrete, espresso supercritical extraction extract (water-soluble), cocoa extract, coffee tincture, angelica pubescens tincture, Pandan extract, vanilla extract, Labdanum concrete, orris oil or concrete, jasmine concrete, tree moss concrete (amber fragrance), tamarind extract, Zimbabwe tobacco extract, tobacco essential oil, burley tobacco extract, flue-cured tobacco absolute A, flue-cured tobacco top note spice extract, and sun-dried top note spice extract.

In some implementations, the aerosol-forming substrate does not comprise nicotine and/or nicotine salts.

A second aspect of this application provides an aerosol generation system. The aerosol generation system includes an electronic vaporization device and the aerosol-forming substrate. The electronic vaporization device is configured to heat and vaporize the aerosol-forming substrate to generate an aerosol.

In the aerosol-forming substrate containing yohimbine or a pharmaceutically acceptable salt of yohimbine or yohimbine hydrate provided in this application, no carbonization occurs after repeated heating by using an electronic vaporization device, and a conversion rate of yohimbine is relatively high, so that a content of yohimbine is close to a dosage through oral administration or injection in current clinical trials, thereby guaranteeing safety.

## BRIEF DESCRIPTION OF THE DRAWINGS

FIG. 1 is a schematic diagram of HPLC detection results according to an implementation of this application;
FIG. 2 is a schematic diagram of a vaporizer in an electronic vaporization device before a carbonization experiment according to an implementation of this application; and
FIG. 3 is a schematic diagram of a vaporizer in an electronic vaporization device after a carbonization experiment according to an implementation of this application.

## DETAILED DESCRIPTION

This application is to be further described with reference to the following embodiments.

Implementation I:

Implementation I of this application provides an aerosol-forming substrate. The aerosol-forming substrate includes yohimbine or a pharmaceutically acceptable salt of yohimbine or yohimbine hydrate.

Based on a total mass of the aerosol-forming substrate, a mass percent of the yohimbine or the pharmaceutically acceptable salt of the yohimbine or the yohimbine hydrate is in a range of 0.1%-10%, preferably 0.1%-8%, further preferably 0.1%-6%, further preferably 0.1%-5%, further preferably 0.5%-5%, further preferably 1%-5%, and further preferably 2%-5%.

In some implementations, the pharmaceutically acceptable salt is selected from at least one of the following: hydrochloride, hydrobromide, benzoate, hydrofluoride, sulfate, nitrate, phosphate, formate, acetate, propionate, oxalate, malonate, succinate, fumarate, maleate, lactate, malate, tartrate, citrate, picrate, mesylate, esilate, benzene sulfonate, aspartate, and glutamate.

In some implementations, the pharmaceutically acceptable salt includes benzoate.

In some implementations, the aerosol-forming substrate further includes a solvent, where the solvent is selected from at least one of the following:

propanediol, butanediol, pentanediol, hexanediol, 1,2,4-butanetriol, dipropylene glycol, diethylene glycol, triethylene glycol, polyethylene glycol 200, polyethylene glycol 400, glycerol triacetate, glycerol, dipropylene glycol ether, ethanol, water, triethyl citrate, caprylic capric triglyceride, isopropanol, sweet orange oil, lemon oil, peppermint oil, palm oil, peanut oil, corn oil, salad oil, xylitol, sorbitol, and erythritol.

In the implementation, propanediol includes 1,2-propanediol and 1,3-propanediol. Butanediol includes 1,2-butanediol, 1,3-butanediol, 1,4-butanediol, and 2,3-butanediol. Pentanediol includes 1,2-pentanediol, 1,4-pentanediol, 2,4-pentanediol, and 1,5-pentanediol. Hexylene glycol includes 1,2-hexanediol, 1,6-hexanediol, and 2,5-hexanediol.

In some implementations, the solvent includes propanediol, and a mass percent of the propanediol in the aerosol-forming substrate is in a range of 20%-60%, preferably 20%-50%, further preferably 20%-45%, further preferably 25%-45%, further preferably 30%-45%, and further preferably 35%-45%.

In some implementations, the solvent includes glycerol, and a mass percent of the glycerol in the aerosol-forming substrate is in a range of 10-60%, preferably 10%-50%, further preferably 10%-40%, further preferably 20%-40%, and further preferably 30%-40%.

In some implementations, a mass ratio of the propanediol to the glycerol is greater than 1, preferably 1.1-4:1, and further preferably 2-4:1.

In some implementations, the aerosol-forming substrate further includes a flavoring agent, and a mass percent of the flavoring agent in the aerosol-forming substrate is in a range of 1%-20%, preferably 5%-20%, further preferably 10%-20%, and further preferably 15%-20%.

In some implementations, the flavoring agent is selected from at least one of the following: nerol, trans-2-hexenol, linalool, benzyl alcohol, 1-hexanol, leaf alcohol, alpha-terpineol, citronellol, beta-phenylethyl alcohol, linalool oxide, geraniol, isoamyl alcohol, octanol, hexanol, decyl alcohol, cinnamyl alcohol, heptanol, eugenol, maltol, ethyl maltol, thymol, isoeugenol, 2-methylbutyric acid, malic acid, n-valeric acid, hexanoic acid, edible acetic acid, n-caprylic acid, strawberry acid, butyric acid, citric acid, propionic acid, 3-methylvaleric acid, isovaleric acid, isoamyl acetate, amyl formate, geranyl formate, butyl formate, benzyl formate, cis-3-Hexenyl formate, gamma-Decanolactone, delta-Nonalactone, gamma-Octalactone, gamma-Heptalactone, gamma-Unsecalactone, delta-Dodecalactone, benzaldehyde, strawberry aldehyde, cinnamyl aldehyde, furfural, citral, acetaldehyde, 3-(Methylthio)propionaldehydel, 3-Mercapto-2-methylpentanal, isobutyraldehyde, trans-2-Octenal, trans-2-Nonenal, trans-2-Decenal, trans,trans-2,4-heptadienal, 2,5-Dimethyl pyrazine, 2-Acetylfuran, 2-Ethyl-3,(5 or 6)-dimethylpyrazine, 2,3,5,6-Tetramethylpyrazine, 2,3,5-Trimethylpyrazine, 2-acetyl pyrazine, acetophenone, beta-ionone, Damascenone 2 (Firmenich), butanedione, alpha-ionone , acetoin (acetyl methyl carbinol), acetoin (acetyl methyl alcohol), methyl heptenone, vanillin, ethyl vanillin, dihydrocoumarin, raspberry ketone, anisole, cedryl methyl ether, methyl 2-methyl-3-furyl disulfide, wintergreen oil, clove bud oil, 10-fold sweet orange oil, rosewood oil, geranium oil, bitter almond oil, clove basil oil, ethyl vanillin, dihydrocoumarin, raspberry ketone, trans-2-ethoxy-5-(1-propenyl)phenol, tolu concrete, Peru concrete, oak concrete, espresso supercritical extraction extract (water-soluble), cocoa extract, coffee tincture, angelica pubescens tincture, Pandan extract, vanilla extract, Labdanum concrete, orris oil or concrete, jasmine concrete, tree moss concrete (amber fragrance), tamarind extract, Zimbabwe tobacco extract, tobacco essential oil, burley tobacco extract, flue-cured tobacco absolute A, flue-cured tobacco top note spice extract, and sun-dried top note spice extract.

It should be noted that, the flavoring agent is not limited to substances listed above, and any flavoring agent that conforms to FEMA coding and CAS coding is applicable.

In some implementations, the aerosol-forming substrate does not comprise nicotine and/or nicotine salts.

I. HPLC test:

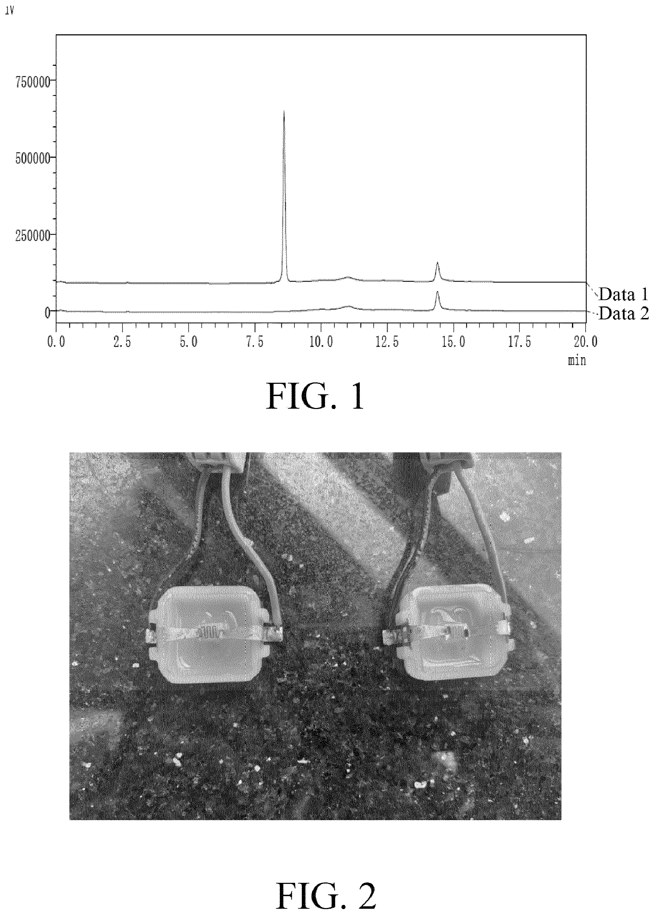

According to the items and parameters shown in the following table, samples with yohimbine and samples without yohimbine are respectively tested through HPLC, and test results may be shown in FIG. 1. Data 1 in the figure is a schematic chromatogram of a sample with yohimbine, and data 2 is a schematic chromatogram of a sample without yohimbine.

| Item | Parameter |
|---|---|
| Chromatographic column | InertSustain C18, 4.6x250 mm, 3.5 $\mu$m |
| Mobile phase | Acetonitrile-water = 40: 60 |
| Test wavelength | 278 nm |
| Flow rate | 1.0 ml/min |
| Column temperature | 40°C |

II. Carbonization experiment

The aerosol-forming substrate of this embodiment is heated (heated for 3s every 27s) by using an electronic vaporization device with a ceramic vaporizer, and a number of heating (or puffs) is 400 times. The aerosol-forming substrate in this embodiment is composed of 1.5% of yohimbine, 40% of propanediol, 37% of glycerol, and 20% of the flavoring agent.

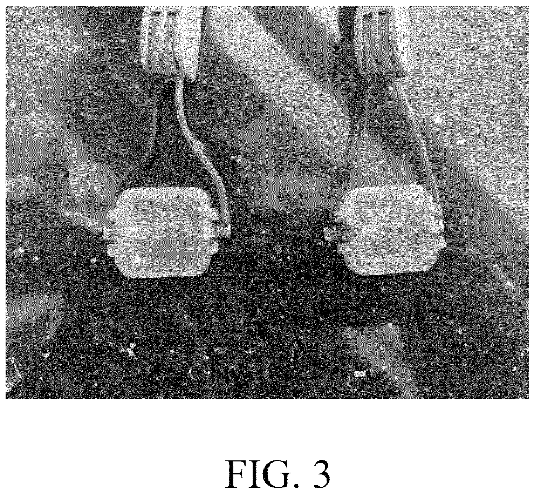

FIG. 2 is a schematic diagram of a vaporizer before the electronic vaporization device heats the aerosol-forming substrate. FIG. 3 is a schematic diagram of a vaporizer after the electronic vaporization device heats the aerosol-forming substrate. Through observation with naked eyes, it can be seen that no gelatinization and carbon deposition occurs in the vaporizer. During the experiment, the experimenter does not notice the odor during inhalation.

During the experiment, an intake of TPM each time is about 5-6 mg. 4% of yohimbine is used as an example. A content of yohimbine each time is about 0.24 mg. It may be learned by looking up relevant information that the commercially available yohimbine tablets (taken orally) are 5.4 mg each time, and assuming its bioavailability is 50%, the intake is about 2.7 mg each time. Therefore, an intake of 10 puffs of a user through the electronic vaporization device is about 2.4 mg (the bioavailability of vaporization intake is much higher than that of oral administration), which is close to the dosage of oral administration in current clinical trials, and the safety can be guaranteed.

III. Conversion rate experiment

Experimental test method:

Embodiment 1: The aerosol-forming substrate is composed of 10 wt% yohimbine, 40 wt% propanediol, and 37 wt% glycerol, and the balance is a flavoring agent.
Embodiment 2: The aerosol-forming substrate is composed of 8 wt% yohimbine, 40 wt% propanediol, and 37 wt% glycerol, and the balance is a flavoring agent.
Embodiment 3: The aerosol-forming substrate is composed of 6 wt% yohimbine, 40 wt% propanediol, and 37 wt% glycerol, and the balance is a flavoring agent.
Embodiment 4: The aerosol-forming substrate is composed of 4 wt% yohimbine, 40 wt% propanediol, and 37 wt% glycerol, and the balance is a flavoring agent.
Embodiment 5: The aerosol-forming substrate is composed of 3 wt% yohimbine, 40 wt% propanediol, and 37 wt% glycerol, and the balance is a flavoring agent.
Embodiment 6: The aerosol-forming substrate is composed of 1.5 wt% yohimbine, 40 wt% propanediol, and 37 wt% glycerol, and the balance is a flavoring agent.
Embodiment 7: The aerosol-forming substrate is composed of 0.5 wt% yohimbine, 40 wt% propanediol, and 37 wt% glycerol, and the balance is a flavoring agent.
Embodiment 8: The aerosol-forming substrate is composed of 0.1 wt% yohimbine, 40 wt% propanediol, and 37 wt% glycerol, and the balance is a flavoring agent.
Embodiment 9: 3 wt% yohimbine is selected as the aerosol-forming substrate, and a mixture of propanediol and glycerol with a mass ratio of about 7: 3 is selected as the solvent.
Embodiment 10: 3 wt% yohimbine is selected as the aerosol-forming substrate, and a mixture of propanediol and glycerol with a mass ratio of about 3: 7 is selected as the solvent.
Embodiment 11: 3 wt% pharmaceutically acceptable benzoate of yohimbine is selected as the aerosol-forming substrate, and a mixture of glycerol and propanediol with a mass ratio of about 1: 1 is selected as the solvent. The pharmaceutically acceptable benzoate of yohimbine may be manufactured by mixing yohimbine alkaloid with benzoic acid.

Embodiment 12: 3 wt% pharmaceutically acceptable hydrochloride of yohimbine is selected as the aerosol-forming substrate, and a mixture of glycerol and propanediol with a mass ratio of about 1: 1 is selected as the solvent. The pharmaceutically acceptable hydrochloride of yohimbine may be manufactured by separation and purification from the bark of the Rubiaceae Corunant Yohimbine tree, or may be obtained through the existing chemical synthesis method, and may further be purchased from the market.

Embodiment 1 to Embodiment 12 are inhaled by using a smoking machine according to international standards, and the aerosol vaporized by the aerosol-forming substrate is collected by using a Cambridge filter, then extracted by using an organic solvent, filtered, and analyzed and calculated through HPLC.

The conversion rate is calculated as follows. A weight difference $m\_0$ of the Cambridge (that is, the weight of the collected aerosol) is calculated. An aerosol-forming substrate mi with the same weight is obtained for extraction by using an organic solvent with the same volume, and then an extract obtained is subjected to HPLC. A ratio of a chromatographic peak area $A_1$ to a concentration Ci of the aerosol-forming substrate in the HPLC is multiplied by a chromatographic peak area $A_0$ of the aerosol, and then the product is divided by the concentration $C_0$ of the aerosol, to obtain the conversion rate. The specific calculation formula is as follows:

$$w\% = \frac{C_1 \times A_0}{A_1 \times C_0} \times 100\%, \text{ where } C_0 = {m_0}/{v}, \text{ and } C_1 = {m_1}/{v}.$$

The calculation of the pharmaceutically acceptable salt of yohimbine is similar to above.

Different electronic vaporization devices are used for inhalation. An electronic vaporization device 1 adopts a vaporizer with a cotton core structure, an electronic vaporization device 2 adopts a vaporizer with a ceramic structure, and an electronic vaporization device 3 adopts a vaporizer with a cotton-covered ceramic structure. The vaporizer with the above structure may be learned from the prior art, and the details are not described herein.

For international standards involved, reference may be made to:

CORESTA RECOMMENDED METHOD N° 81, Afnor standardization XP D90-300-3, International Standard ISO 20768:2018, and PD CEN/TR 17236:2018.

| Puff duration | 3.0s±0.1s |
|---|---|
| Puff volume | 55 mL±0.3 mL |
| Puff frequency | 30s±0.5s |
| Number of puffs of each group | 20 |
| Group interval time | 300s±120s |
| Maximum flow | 18.5 mL/s±0.1 mL/s |
| Pressure drop | <50hPa |
| Group | 5 |
| Total number of puffs | 100 |
| Total duration of vaporization | 300s |

Experimental test results: Different electronic vaporization devices are used for puffing, and corresponding conversion rates (k1-k3) thereof are calculated respectively. An average of the conversion rates is calculated based on the conversion rates corresponding to different electronic vaporization devices. The results are shown in the following table:

| Embodiment | Conversion rate k1 by using the electronic vaporization device 1 | Conversion rate k2 by using the electronic vaporization device 2 | Conversion rate k3 by using the electronic vaporization device 3 | Average |
|---|---|---|---|---|
| 1 | 31% | 40% | 30% | 34% |
| 2 | 37% | 45% | 36% | 39% |
| 3 | 41% | 50% | 40% | 44% |

(continued)

| Embodiment | Conversion rate k1 by using the electronic vaporization device 1 | Conversion rate k2 by using the electronic vaporization device 2 | Conversion rate k3 by using the electronic vaporization device 3 | Average |
|---|---|---|---|---|
| 4 | 45% | 52% | 43% | 47% |
| 5 | 48% | 55% | 49% | 51% |
| 6 | 56% | 68% | 58% | 61% |
| 7 | 65% | 72% | 66% | 68% |
| 8 | 68% | 75% | 72% | 72% |
| 9 | 67% | 78% | 55% | 67% |
| 10 | 45% | 55% | 48% | 49% |
| 11 | 62% | 78% | 59% | 66% |
| 12 | 46% | 65% | 60% | 57% |

It can be seen from the above experimental test results:

1) In Embodiment 1 to Embodiment 8, with the increase of the content of yohimbine in the aerosol-forming substrate, the conversion rate (average value) tends to decline. When the content of yohimbine in the aerosol-forming substrate reaches 10%, the conversion rate (average value) is about 34%.

2) In Embodiment 5 and Embodiment 9 to Embodiment 10, the content of propanediol in the solvent has great influence on the conversion rate. When the mass ratio of propanediol to glycerol is 7:3, the conversion rate (average value) can reach 67%, which is higher than the conversion rate in Embodiment 5 or Embodiment 10.

3) In Embodiment 5 and Embodiment 11 to Embodiment 12, the conversion rate (average value) of the pharmaceutically acceptable salt of yohimbine is generally high, and the conversion rate in Embodiment 11 or Example 12 is higher than the conversion rate in Embodiment 5. Yohimbine is a pharmaceutically acceptable benzoate, and the conversion rate (average value) may reach 66%.

Implementation II:

Implementation II of this application provides an aerosol generation system, including an electronic vaporization device and the aerosol-forming substrate described in Implementation I.

The electronic vaporization device is configured to heat and vaporize the aerosol-forming substrate to generate an aerosol.

This written description uses examples to disclose this application, including the best mode, and also to enable any person skilled in the art to manufacture and use this application. The patentable scope of this application is defined by the claims, and may include another example that occur to a person skilled in the art. Such other examples are intended to be within the scope of the claims if they have structural elements that do not differ from the literal language of the claims, or if they include equivalent structural elements with insubstantial differences from the literal language of the claims. To the extent that no inconsistency arises, all citations referenced herein are incorporated herein by reference.

**Claims**

1.    An aerosol-forming substrate, comprising yohimbine or a pharmaceutically acceptable salt of yohimbine or yohimbine hydrate, wherein
based on a total mass of the aerosol-forming substrate, a mass percent of the yohimbine or the pharmaceutically acceptable salt of the yohimbine or the yohimbine hydrate is in a range of 0.1%-10%, preferably 0.1%-8%, further preferably 0.1%-6%, further preferably 0.1%-5%, further preferably 0.5%-5%, further preferably 1%-5%, and further preferably 2%-5%.

2.    The aerosol-forming substrate according to claim 1, wherein the pharmaceutically acceptable salt is selected from at least one of the following:

hydrochloride, hydrobromide, benzoate, hydrofluoride, sulfate, nitrate, phosphate, formate, acetate, propionate, oxalate, malonate, succinate, fumarate, maleate, lactate, malate, tartrate, citrate, picrate, mesylate, esilate, benzene sulfonate, aspartate, and glutamate.

3. The aerosol-forming substrate according to claim 2, wherein the pharmaceutically acceptable salt comprises benzoate.

4. The aerosol-forming substrate according to any of claims 1 to 3, further comprising a solvent, wherein the solvent is selected from at least one of the following:
propanediol, butanediol, pentanediol, hexanediol, 1,2,4-butanetriol, dipropylene glycol, diethylene glycol, triethylene glycol, polyethylene glycol 200, polyethylene glycol 400, glycerol triacetate, glycerol, dipropylene glycol ether, ethanol, water, triethyl citrate, caprylic capric triglyceride, isopropanol, sweet orange oil, lemon oil, peppermint oil, palm oil, peanut oil, corn oil, salad oil, xylitol, sorbitol, and erythritol.

5. The aerosol-forming substrate according to claim 4, wherein the solvent comprises propanediol, and a mass percent of the propanediol in the aerosol-forming substrate is in a range of 20%-60%, preferably 20%-50%, further preferably 20%-45%, further preferably 25%-45%, further preferably 30%-45%, and further preferably 35%-45%.

6. The aerosol-forming substrate according to claim 5, wherein the solvent comprises glycerol, and a mass percent of the glycerol in the aerosol-forming substrate is in a range of 10-60%, preferably 10%-50%, further preferably 10%-40%, further preferably 20%-40%, and further preferably 30%-40%.

7. The aerosol-forming substrate according to claim 6, wherein a mass ratio of the propanediol to the glycerol is greater than 1, preferably 1.1-4:1, and further preferably 2-4:1.

8. The aerosol-forming substrate according to any of claims 1 to 7, further comprising a flavoring agent.

9. The aerosol-forming substrate according to any of claims 1 to 8, wherein the aerosol-forming substrate does not comprise nicotine and/or nicotine salts.

10. An aerosol generation system, comprising an electronic vaporization device and the aerosol-forming substrate according to any of claims 1 to 9, wherein
the electronic vaporization device is configured to vaporize the aerosol-forming substrate to generate an aerosol.

FIG. 1

FIG. 2

FIG. 3

## INTERNATIONAL SEARCH REPORT

| International application No. |
| --- |
| **PCT/CN2022/086384** |

| A. | CLASSIFICATION OF SUBJECT MATTER |
| --- | --- |

A61K 9/12(2006.01)i; A61M 11/00(2006.01)i

According to International Patent Classification (IPC) or to both national classification and IPC

| B. | FIELDS SEARCHED |
| --- | --- |

Minimum documentation searched (classification system followed by classification symbols)

A61K9/-, A61M11/-

Documentation searched other than minimum documentation to the extent that such documents are included in the fields searched

Electronic data base consulted during the international search (name of data base and, where practicable, search terms used)

CNTXT, ENTXTC, CNKI: 合元科技, 育亨宾, 气溶胶, 气胶, 烟雾质, 雾化, 溶剂, 丙二醇, 甘油, 丙三醇; WPABS, ENTXT, VEN, WEB OF SCIENCE: aphrodine, corynine, yohimbime, yohimbine, aphrodyne, aerosol, pulverization, atomization, solvent, propylene glycol, glycerol, glycerin.

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2011074015 A2 (THEMIS MEDICARE LTD et al.) 23 June 2011 (2011-06-23) description page 8 paragraph 2, page 9 paragraph 4, page 21 paragraph 8- page 22 paragraph 1, page 25 paragraph 3 | 1-5, 8-10 |
| A | CN 1276715 A (THE BOARD F REGENTS OF THE UNIV. OF OKLAHOMA) 13 December 2000 (2000-12-13) entire document | 1-10 |
| A | US 6110448 A (THE BOARD OF REGENTS OF THE UNIVERSITY OF OKLAHOMA) 29 August 2000 (2000-08-29) entire document | 1-10 |
| A | US 2005025844 A1 (Matthias Boldt) 03 February 2005 (2005-02-03) entire document | 1-10 |
| A | US 7470703 B1 (UNIV TENNESSEE RES FOUNDATION et al.) 30 December 2008 (2008-12-30) entire document | 1-10 |
| A | CA 3021459 A1 (SPRINGER JOHN S) 19 April 2020 (2020-04-19) entire document | 1-10 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **28 June 2022** | **08 July 2022** |

| Name and mailing address of the ISA/CN | Authorized officer |
| --- | --- |
| **China National Intellectual Property Administration (ISA/ CN)** No. 6, Xitucheng Road, Jimenqiao, Haidian District, Beijing 100088, China | |
| Facsimile No. **(86-10)62019451** | Telephone No. |

Form PCT/ISA/210 (second sheet) (January 2015)

**INTERNATIONAL SEARCH REPORT**
Information on patent family members

| International application No. |
|---|
| **PCT/CN2022/086384** |

| Patent document cited in search report | | | Publication date (day/month/year) | Patent family member(s) | | | Publication date (day/month/year) |
|---|---|---|---|---|---|---|---|
| WO | 2011074015 | A2 | 23 June 2011 | None | | | |
| CN | 1276715 | A | 13 December 2000 | JP | 2001517612 | A | 09 October 2001 |
| | | | | CN | 1515242 | A | 28 July 2004 |
| | | | | US | 6096295 | A | 01 August 2000 |
| | | | | AU | 8490198 | A | 12 April 1999 |
| | | | | CA | 2304452 | A1 | 01 April 1999 |
| | | | | KR | 20010024317 | A | 26 March 2001 |
| | | | | WO | 9915149 | A1 | 01 April 1999 |
| | | | | US | 5879665 | A | 09 March 1999 |
| US | 6110448 | A | 29 August 2000 | None | | | |
| US | 2005025844 | A1 | 03 February 2005 | None | | | |
| US | 7470703 | B1 | 30 December 2008 | None | | | |
| CA | 3021459 | A1 | 19 April 2020 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110398742
- CN 112155255 A